(19) Europäisches Patentamt

European Patent Office

Office européen des brevets

(11) **EP 2 803 998 B1**

(12) **EUROPEAN PATENT SPECIFICATION**

(45) Date of publication and mention
of the grant of the patent:
**27.11.2019 Bulletin 2019/48**

(21) Application number: **13736058.2**

(22) Date of filing: **10.01.2013**

(51) Int Cl.:
*G01N 33/49* (2006.01)     *G01N 33/48* (2006.01)
*G01N 1/40* (2006.01)      *G01N 15/05* (2006.01)
*G01N 15/14* (2006.01)

(86) International application number:
**PCT/JP2013/050329**

(87) International publication number:
**WO 2013/105612 (18.07.2013 Gazette 2013/29)**

(54) **METHOD FOR EVALUATING SYSTEM FOR QUANTIFYING CELL OF INTEREST IN BLOOD**

VERFAHREN ZUR BEWERTUNG EINES SYSTEMS ZUR QUANTIFIZIERUNG BESTIMMTER ZELLEN IM BLUT

PROCÉDÉ D'ÉVALUATION D'UN SYSTÈME POUR QUANTIFIER UNE CELLULE INTÉRESSANTE DANS LE SANG

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **13.01.2012 JP 2012005079**

(43) Date of publication of application:
**19.11.2014 Bulletin 2014/47**

(73) Proprietor: **Konica Minolta, Inc.**
**Tokyo 100-7015 (JP)**

(72) Inventors:
• **ARAKI, Jungo**
**Tokyo 100-7015 (JP)**
• **CHIYODA, Tsuneko**
**Tokyo 100-7015 (JP)**
• **MIYAZAKI, Koji**
**Tokyo 100-7015 (JP)**

(74) Representative: **Gille Hrabal**
**Brucknerstrasse 20**
**40593 Düsseldorf (DE)**

(56) References cited:
EP-A1- 0 958 046        WO-A1-97/38313
JP-A- 2000 502 892      JP-A- 2004 534 210
JP-A- 2009 288 254      US-A- 4 774 965
US-A- 5 627 037         US-A- 5 840 502
US-A1- 2002 090 741     US-A1- 2010 248 257

• YABUSAKI K ET AL: "HIGH-SENSITIVITY
METHOD FOR COUNTING LOW
CONCENTRATIONS OF PARTICLES IN LIQUIDS
USING CENTRIFUGATION AND IMAGE
ANALYSIS", JAPANESE JOURNAL OF APPLIED
PHYSICS, JAPAN SOCIETY OF APPLIED
PHYSICS, JP, vol. 39, no. 6A, PART 01, 1 June
2000 (2000-06-01), pages 3641-3644,
XP001014831, ISSN: 0021-4922, DOI:
10.1143/JJAP.39.3641

## Description

TECHNICAL FIELD

The present invention relates to a method for evaluating the reliability of a system for quantifying cells of interest.

BACKGROUND ART

Circulating tumor cells (CTCs), circulating stem cells, circulating endothelial cells and the like (hereinafter also collectively referred to as "rare cells") are cells that are extremely rarely present in whole blood depending on pathological conditions. Although detection of such rare cells is clinically obviously useful, detection of all rare cells in a whole-blood sample is still difficult.

In recent years, detection of rare cells by use of various cell separation methods has been attempted, and products for such detection are commercially available. However, in any of these, validity (whether rare cells are lost or not, and whether contamination with unnecessary cells occurs or not), and a means for securing of the detection results are considered to be important due to rareness of target cells.

Since the detection speed of optical detectors has increased, a method has been proposed in which rare cells are separated together with leukocytes by specific-gravity separation such as density gradient centrifugation, followed by detection of all cells. In this method, a solution of a polymer having a predetermined specific gravity (i.e., separation medium) such as Ficoll (registered trademark of GE Healthcare Japan) is used. After separating erythrocytes into a layer lower than, and rare cells and leukocytes into a layer upper than, the separation medium, the extracted rare cells and leukocytes are scanned with an optical detector to identify the rare cells contained therein.

However, the densities of separation media such as Ficoll may change depending on the ambient environment (for example, temperature), and this may result in failure to achieve desired separation, decreasing the reliability of the detection result. Further, in steps other than the separation, underestimation of the number of rare cells due to loss of the cells, or inaccurate calibration of the detector due to aged deterioration, may be missed. This may cause an error in counting of the number of rare cells and hence lead to decreased reliability.

In view of this, use of the stabilized cell described in Patent Document 1 as an internal control in a method of isolation and identification of rare cells has been proposed. In cases where the rare cells are CTCs, this stabilized cell can be produced by, for example, fluorescently labeling a breast cancer cell line SKBR-3 and then fixing the SKBR-3 cell with paraformaldehyde or the like.

However, since the stabilized cell as an internal control is produced by special treatment of a live cell, its specific gravity may be largely different from that of the rare cells. Therefore, desired separation cannot be achieved in some cases.

Patent Document 2 discloses a method in which organosilanized colloidal silica (OCS) particles are added into a sample and subsequently a density gradient centrifugation is carried out to enrich rare cells at interface and red blood cells in pellet.

Patent Document 3 discloses the use of microparticles as supports for antibodies, which can bind to non-rare cells in a sample and enable separation of the non-rare cells from rare cells in the sample. Nevertheless, density gradient centrifugation is not performed in the presence of microparticles according to this method.

US 2010/248257 A1 discloses a method of correcting the number of rare cells after being separated from the non-rare cells via density gradient centrifugation. A known number of particles, which have a specific gravity close to the specific gravity of the rare cells, is added to the blood sample. Their number is counted in the separated layer and used to correct the number of rare cells.

CITATION LIST

[Patent Documents]

[Patent Document 1] Japanese Unexamined Patent Application Publication (Translation of PCT Application) No. 2004-534210
[Patent Document 2] European Patent Publication EP 0 958 046 A1
[Patent Document 3] International Patent Application WO 97/38313 A1

SUMMARY OF THE INVENTION

PROBLEMS TO BE SOLVED BY THE INVENTION

It is an object of the present invention to provide an evaluation method for evaluating the reliability of a system for quantifying a cell of interest according to claim 1.

MEANS FOR SOLVING THE PROBLEMS

The evaluation method of the present invention is a method for evaluating reliability of a system for quantifying a cell(s) of interest that is/are potentially contained in a blood-derived sample and has/have a specific gravity larger than 1.025 but smaller than 1.120; the method comprising the Steps (a) to (d) below.

(a) Separating a blood-derived sample containing a known number (P0) of resin particles P having a specific gravity larger than 1.025 but smaller than 1.120 and a known number (N0) of resin particles N having a specific gravity of not less than 1.090 and not more than 1.120 by density gradient centrifugation into at least two layers including a layer containing a larger amount of erythrocytes and a layer containing a larger amount of cells other than erythrocytes;

(b) extracting the layer containing a larger amount of cells other than erythrocytes and counting the number (P1) of resin particles P and the number (N1) of resin particles N contained in the layer;

(c) calculating (P1/P0)×100 and comparing the calculated value with a predetermined reference value to evaluate the extent to which the whole system functions; and

(d) calculating N1/P1 and comparing the calculated value with a predetermined reference value to evaluate the extent to which the layers are separated by the density gradient centrifugation.

The resin particles N are preferably formed from a water-insoluble resin and labeled with an optically detectable dye Db having an emission wavelength different from the emission wavelength of the optically detectable dye Da.

The evaluation method of the present invention preferably further comprises the step of:

(e) measuring an emission signal derived from the resin particles P in the number of P1 or the resin particles N in the number of N1, and calibrating an emission signal detector.

An example of a kit, not part of the present invention, includes: a kit to be used for the evaluation method of the present invention, comprising known numbers of the resin particles P and the resin particles N.

EFFECT OF THE INVENTION

The present invention enables detection of errors in the steps (especially the density gradient centrifugation step) of clinical tests or the like.

BRIEF DESCRIPTION OF THE DRAWINGS

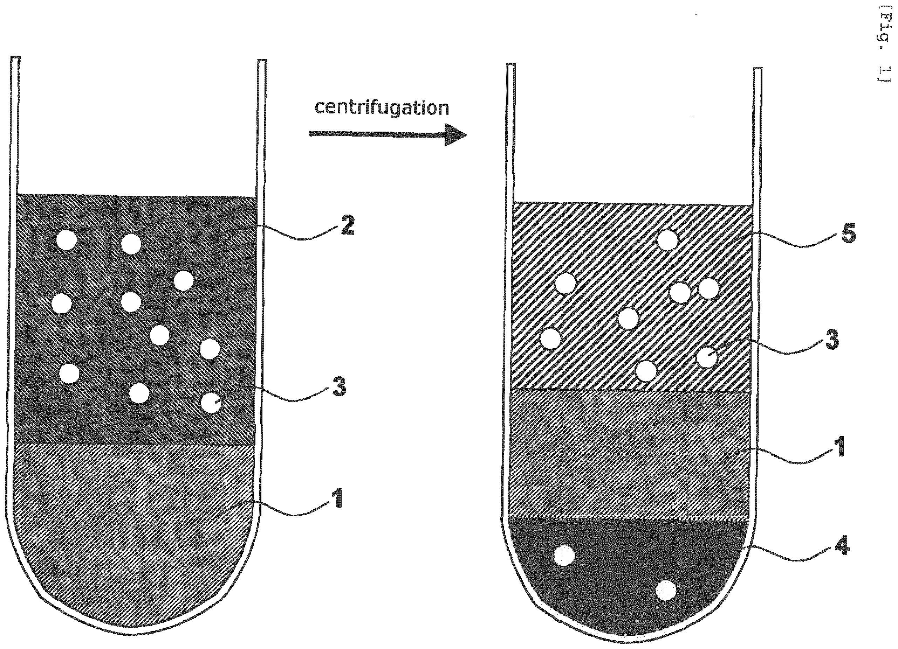

[Fig. 1] Fig. 1 is a schematic diagram showing the blood-derived sample before and after application of density gradient centrifugation.

DESCRIPTION OF EMBODIMENTS

A quantification method, not part of the present invention, the evaluation method according to the present invention and the kit, not part of the present invention, are specifically described below.

<Quantification Method>

The quantification method is a method for quantifying a cell(s) of interest that is/are potentially contained in a blood-derived sample and has/have a specific gravity larger than a specific gravity of blood plasma but smaller than a specific gravity of erythrocytes, the method including the Steps (A) to (C) below.

(A) Separating a blood-derived sample containing a known number (P0) of resin particles P having a specific gravity larger than a specific gravity of blood plasma but smaller than a specific gravity of erythrocytes by density gradient centrifugation into at least two layers including a layer containing a larger amount of erythrocytes and a layer containing a larger amount of cells other than erythrocytes;

(B) extracting the layer containing a larger amount of cells other than erythrocytes and counting the number of cell(s) of interest and the number (P1) of the resin particles contained in the layer; and

(C) correcting the number of cell(s) of interest by multiplying the number of cell(s) of interest by P0/P1.

In the quantification method, the "cell of interest" means the cell that is to be quantified by the quantification method.

The quantification method is described in more detail by using Fig. 1.

[Step (A)]

Step (A) is a step of separating a blood-derived sample containing a known number (P0) of resin particles P having a specific gravity larger than a specific gravity of blood plasma but smaller than a specific gravity of erythrocytes by density gradient centrifugation into at least two layers including a layer containing a larger amount of erythrocytes and a layer containing a larger amount of cells other than erythrocytes. The resin particle P in the present invention functions as an internal standard as described below in the section "Resin Particle P".

As illustrated in Fig. 1, when density gradient centrifugation using a specific-gravity liquid (1) is carried out for a blood-derived sample (2) containing resin particles P (3) in the number of P0 (for example, 10, as shown in Fig. 1) having a specific gravity larger than a specific gravity of blood plasma but smaller than a specific gravity of erythrocytes, a layer (4) containing a larger amount of erythrocytes, which are cells having the largest specific gravity, and a layer (5) containing a larger amount of cells other than erythrocytes are separated from each other by the layer of the specific-gravity liquid (1). Examples of the "cells other than erythrocytes" include platelets and leukocytes, and cells of interest such as CTCs.

As the density gradient centrifugation used in the present method, methods may be used which are described below in the section "Density Gradient Centrifugation", and, as the specific-gravity liquid (1), separation liquids or separation media may be used which are described below in the section "Density Gradient Centrifugation".

A more specific procedure is as follows. For example, as shown in the left half of Fig. 1, a specific-gravity liquid (1) having a known specific gravity is placed in a centrifuge tube. On the specific-gravity liquid (1), a blood-derived sample (2) containing a known number (P0) of resin particles P (3) having a specific gravity larger than a specific gravity of blood plasma but smaller than a specific gravity of erythrocytes is placed, followed by performing centrifugation. By this, Step (A) can be carried out. By such centrifugation, the content of the centrifuge tube is separated, as shown in the right half of Fig. 1, along the density gradient into a layer (4) containing a larger amount of erythrocytes, the specific-gravity liquid (1), and a layer (5) containing a larger amount of cells other than erythrocytes.

[Step (B)]

Step (B) is a step of extracting the layer containing a larger amount of cells other than erythrocytes and counting the number of cells of interest and the number (P1) of resin particles P contained in the layer.

A purpose of counting the number (P1) of resin particles P in addition to the number of the cells of interest in the "layer containing a larger amount of cells other than erythrocytes" is to presume the number of cells of interest that are potentially contained in the blood-derived sample based on the ratio of the number (P1) of resin particles P found in the "layer containing a larger amount of cells other than erythrocytes" to the number (P1) of resin particles P added to the blood-derived sample.

As illustrated in Fig. 1, in Step (B), the number of cells, among the cells of interest that are potentially contained in the blood-derived sample (2), that could be extracted into the "layer (5) containing a larger amount of cells other than erythrocytes", and the number of resin particles (P) (3) that could be extracted into the "layer (5) containing a larger amount of cells other than erythrocytes" can be obtained by counting by a certain known method. By this, it can be seen that the layer (5) contains the resin particles P (3) in the number of P1 (for example, 8, as shown in Fig. 1).

A specific method of counting the number of cells of interest and the number (P1) of resin particles P is described below in the section "Method of Counting Number of Cells of Interest or Number of Resin Particles".

[Step (C)]

Step (C) is a step of correcting the number of cell(s) of interest by multiplying the number of cell(s) of interest counted by Step (B) by P0/P1.

For example, in cases where it can be confirmed that the "layer (5) containing a larger amount of cells other than erythrocytes" obtained by subjecting a blood-derived sample containing cells of interest in the number of T0 and resin particles P in the number of P0 to density gradient centrifugation contains the cells of interest in the number of T1 and the resin particles P in the number of P1, the ratio of T1 to T0 can be considered to be the same as the ratio of P1 to P0. That is, the following relationship:

$$T1/T0 = P1/P0$$

can be considered to be satisfied. Thus, the number T0 of cells of interest contained in the blood-derived sample can be calculated as follows:

$$T0 = T1 \times (P0/P1).$$

In the case shown in Fig. 1, the number of cells of interest contained in the layer (5) (for example, 8) multiplied by P0/P1 (10/8, in the case of Fig. 1) equals 10 ($8 \times 10/8$; Step (C)). From this result, the number of cells of interest contained in the original blood-derived sample (2) can be quantified as 10, and the number of cells of interest lost during the density gradient centrifugation can be assumed to be 2.

That is, the present method enables more accurate quantification of the number of cells of interest contained in the blood-derived sample (2) by correcting the number of cells of interest contained in the layer (5).

Since the principle of density gradient centrifugation is used in the present method, the specific gravity of the resin particles P is preferably close to the specific gravity of the cells of interest, more preferably the same as the specific gravity of the cells of interest. In density gradient centrifugation, each component in a blood-derived sample is separated according to its specific gravity. Therefore, in the present method, the closer the specific gravity of the resin particles P to the specific gravity of the cells of interest, the more accurately quantification of the cells of interest can be carried out. Further, in order to allow sufficient separation of the resin particles P and the cells of interest from erythrocytes, the specific gravity of the specific-gravity liquid is preferably larger than the specific gravities of the resin particles P and the cells of interest, but smaller than the specific gravity of erythrocytes. The relationship among the specific gravities of components in the blood-derived sample and the resin particles P is especially preferably as follows: blood plasma < platelets < leukocytes and cells of interest = resin particles P < specific-gravity liquid < erythrocytes.

[Blood-derived Sample]

Examples of the blood-derived sample used in the present method include blood and other body fluids themselves, and dilutions prepared by diluting these with an appropriate buffer or the like (that is, body fluids and diluted body fluids); and suspensions of tissue-derived cells, cultured cells or the like. Among these, preferred examples of the "blood-derived sample" include blood, and dilutions prepared by diluting blood with an appropriate diluent normally used in the field of the present method such as a buffer; that is, blood and diluted blood.

The "cells of interest" that are potentially contained in a blood-derived sample and to be quantified in the present method are cells having a specific gravity larger than a specific gravity of blood plasma (1.025 to 1.029) but smaller than a specific gravity of erythrocytes (1.090 to 1.120) . Such "cells of interest" are preferably at least one type of rare cells selected from the group consisting of circulating tumor cells (CTCs), circulating stem cells and circulating endothelial cells. CTCs are especially preferred.

[Resin Particle P]

The resin particles P used in the present method are used as an internal control for the cells of interest, and have a specific gravity larger than a specific gravity of blood plasma but smaller than a specific gravity of erythrocytes, preferably a specific gravity of not less than 1.040 and not more than 1.085, more preferably a specific gravity of not less than 1.040 and not more than 1.077. The resin particles P desirably has a specific gravity of not less than 1.040 and not more than the specific gravity of the separation medium used in the density gradient centrifugation.

The resin particles P are substantially spherical, and each particle has a size of not less than that sufficient for maintaining the suspended state in the blood-derived sample but not more than about 100 $\mu$m. More specifically, the average particle diameter is preferably about 0.2 to 20 $\mu$m.

Preferably, the resin particles P are formed from a water-insoluble resin, and do not aggregate. Examples of such a resin include polystyrene, polymethylmethacrylate, polyvinyltoluene and polyacrylate, but the resin is not limited to these in the present method.

The resin particles P are preferably labeled with an optically detectable dye Da such that the number of particles can be easily counted in Step (B) described above and Step (b) in the evaluation method described below. The dye Da used in this process is more preferably a fluorescent dye. The labeling with the dye Da may be carried out in a mode in which the dye is bound or attached on the surface of the particle, or in a mode in which the dye is kneaded in the particle.

In some cases, depending on the type of the resin used as the resin particles P, labeling with the dye Da is not necessary, and the number of resin particles P can be detected by autofluorescence of the resin itself. In such cases, introduction of the dye Da is not necessary.

Examples of the fluorescent dye Da include fluorescent dyes described in JP 2010-169519 A such as fluorescein-based fluorescent dyes, rhodamine-based fluorescent dyes and cyanine-based fluorescent dyes; quinoxaline-based fluorescent dyes; other synthetic fluorescent dyes; and dyes derived from living bodies, such as porphyrin-based dyes and phycobilin-based dyes. Examples of the phycobilin-based dyes include phycoerythrin (PE).

Production of the resin particles P using a water-insoluble resin or a resin that emits autofluorescence can be carried out using various known methods.

As the resin particles P, commercially available products such as the polystyrene particles manufactured by Spherotech, Inc. (specific gravity, 1.050; average particle diameter, 20 $\mu$m) may also be used, or, in cases where the specific gravity can be adjusted, the fluorescent microparticles described in JP H4-252957 A may also be used.

[Density Gradient Centrifugation]

As the density gradient centrifugation in the present method, a conventional method may be used as appropriate. Examples of separation liquids or separation media used for the density gradient centrifugation include Ficoll and Percoll (these are registered trademarks of GE Healthcare Japan), which are commercially available, and sucrose solutions. The separation liquid or separation medium in the present invention is not limited to these as long as it is a separation liquid or separation medium that has a specific gravity with which erythrocytes contained in the blood-derived sample can be separated from other cells, and whose osmotic pressure and pH can be adjusted such that destruction of cells can be avoided.

The separation liquid or separation medium may be a combination of two or more separation liquids or separation media having different specific gravities (for example, specific gravities of 1.077 and 1.119) . Such use of two or more separation liquids or separation media having different specific gravities allows further separation of the "layer containing a larger amount of cells other than erythrocytes", which is preferred in view of further elimination of cells other than the cells of interest.

[Method of Counting Number of Cells of Interest or Number of Resin Particles]

Examples of the method for counting the number of cells of interest in the Step (B) described above include a method in which only the cells of interest are labeled with the dye having an emission wavelength different from the emission wavelength of the optically detectable dye with which the resin particles P are labeled, and the labeled cells are counted under a fluorescence microscope.

Preferred examples of the dye that can be used for labeling the cells of interest include the fluorescent dyes in the section "Resin Particle P" described above.

In the present method, the dye used for labeling the resin particles P or fluorescence of the resin particles P themselves, and the dye used for labeling the cells of interest, may have either the same excitation wavelength or different excitation wavelengths as long as their emission wavelengths are different from each other.

As the method for labeling the cells of interest, various known methods may be used. A preferable example of the method is labeling by utilization of antigen-antibody reaction with an antibody labeled with an appropriate dye. In another method, in cases where the cells of interest have a unique functional group that is not found in other cells, a dye having a reactive functional group that is capable of binding to the unique functional group may be used as the dye, and the dye may be introduced into the cells of interest through various chemical bonds such as the covalent bond or hydrogen bond.

In cases where the cells of interest themselves emit fluorescence, labeling of the cells of interest with a dye is not necessary, and the number of cells of interest may be counted based on the fluorescence from the cells of interest themselves.

As the method for counting the number of cells of interest, various known methods may be used. Preferred examples of such methods include a method in which a liquid containing the cells of interest and the like is spread on a plane, and this plane is two-dimensionally scanned to detect fluorescence from the cells of interest, thereby counting the number of cells. Such a method may be a method in which the cells are counted under a fluorescence microscope, and, for example, the liquid containing the cells of interest may be spread on an appropriate plane such as a cell culture dish, and the plane may be two-dimensionally scanned to count fluorescence from the cells of interest. However, in the present method, the method of counting the number of the cells of interest is not limited to a method by counting under a fluorescence microscope. For example, the plane may be irradiated with an appropriate excitation light, and two-dimensional scanning of the plane may be carried out with an appropriate photoelectric conversion element such as a photomultiplier. The locations of the cells of interest may be evaluated based on the intensities and positions of fluorescence, and the number of cells of interest may be counted based on the results of evaluation. Alternatively, a fluorescence image of the plane irradiated with an appropriate excitation light may be captured with a known image sensor in which a plurality of image sensors are arranged linearly or two-dimensionally, and the number of fluorescent spots distributed in the fluorescence image may be counted to count the number of cells of interest.

In the evaluation method of the present invention described later, the number of cells of interest may be counted by the same method as described above also in cases where the number of cells of interest is counted in addition to the number of resin particles P and the number of resin particles N in the Step (b) described below. In such cases, the dye used for labeling the resin particles P or the fluorescence from the resin particles P themselves; the dye used for labeling the resin particles N or the fluorescence from the resin particles N themselves; and the dye used for labeling the cells of interest; may have either the same excitation wavelength or excitation wavelengths different from each other, as long as they have emission wavelengths different from each other.

Examples of the method for counting the number of resin particles P in the Step (B) described above and the method for counting the numbers of the resin particles P and the resin particles N in the Step (b) described below include flow cytometry, in addition to the above method used for two-dimensionally counting the cells of interest.

The counting of the number of cells of interest, counting of the number of resin particles P, and counting of the number of resin particles N may be carried out either simultaneously or separately. The methods of these counting processes may be either the same as or different from each other. In cases where the counting of the number of cells of interest, counting of the number of resin particles P, and counting of the number of resin particles N are carried out based on fluorescence, all of these counting processes may be carried out either using an excitation light having the same wavelength or using excitation lights having different wavelengths. Further, if necessary, the wavelength of fluorescence may be separated using an appropriate filter.

<Evaluation Method>

The evaluation method of the present invention is a method for evaluating reliability of a system for quantifying a cell(s) of interest that is/are potentially contained in a blood-derived sample and has/have a specific gravity larger than a specific gravity of blood plasma but smaller than a specific gravity of erythrocytes, the method including the Steps (a) to (d) below.

(a) Separating a blood-derived sample containing a known number (P0) of resin particles P having a specific gravity larger than a specific gravity of blood plasma but smaller than a specific gravity of erythrocytes and a known number (N0) of resin particles N having a specific gravity of not less than 1.090 and not more than 1.120 by density gradient centrifugation into at least two layers including a layer containing a larger amount of erythrocytes and a layer containing a larger amount of cells other than erythrocytes;

(b) extracting the layer containing a larger amount of cells other than erythrocytes and counting the number (P1) of resin particles P and the number (N1) of resin particles N contained in the layer;

(c) calculating (P1/P0)×100 and comparing the calculated value with a predetermined reference value to evaluate the extent to which the whole system functions; and

(d) calculating N1/P1 and comparing the calculated value with a predetermined reference value to evaluate the extent to which the layers are separated by the density gradient centrifugation.

The evaluation method of the present invention herein preferably further includes the step of:
(e) measuring an emission signal derived from the resin particles P in the number of P1 or the resin particles N in the number of N1, and calibrating an emission signal detector.

In the evaluation method according to the present invention, the "cells of interest" means the cells to be quantified by the "quantification system" to be evaluated by the evaluation method of the present invention.

[Resin Particle N]

The resin particles N used in the present invention are used as an internal control for erythrocytes, and have almost the same specific gravity as erythrocytes, that is, a specific gravity of not less than 1.090 and not more than 1.120.

The resin particles N are preferably labeled with an optically detectable dye Db having an emission wavelength different from the emission wavelength of the optically detectable dye Da, more preferably an optically detectable fluorescent dye Db having an emission wavelength different from the emission wavelength of the optically detectable dye Da. The mode of labeling is the same as that of the resin particles P described above. Examples of fluorescent dyes that may be used as the fluorescent dye Db include those described above as the fluorescent dye Da.

The resin particle N has a size of not less than that sufficient for maintaining the suspended state in the blood-derived sample but not more than about 100 μm. More specifically, the average particle diameter is preferably about 0.2 to 20 μm.

Examples of the type of the resin that forms the resin particles N and the method of producing the resin particles include those described above in the section "Resin Particle P".

As the resin particle N, commercially available products such as the spherical microparticle made of cross-linked butyl polymethacrylate manufactured by Sekisui Plastics Co., Ltd. (BM30X-5, specific gravity, 1.100; average particle diameter, 5 μm) may be used, and, in cases where the specific gravity can be adjusted to not less than 1.090 and not more than 1.120, the fluorescent microparticles described in JP H4-252957 A may also be used.

[Step (a)]

Step (a) is a step of separating a blood-derived sample containing a known number (P0) of resin particles P having a specific gravity larger than a specific gravity of blood plasma but smaller than a specific gravity of erythrocytes and a known number (N0) of resin particles N having a specific gravity of not less than 1.090 and not more than 1.120 by density gradient centrifugation into at least two layers including a layer containing a larger amount of erythrocytes and a layer containing a larger amount of cells other than erythrocytes.

In the present invention, the Step (a) can be carried out by the same method as the Step (A) except that, in addition to a known number (P0) of resin particles P having a specific gravity larger than a specific gravity of blood plasma but smaller than a specific gravity of erythrocytes, a known number (N0) of resin particles N having a specific gravity of not less than 1.090 and not more than 1.120 are further added to the blood-derived sample to which density gradient centrifugation is applied

[Step (b)]

Step (b) is a step of extracting the layer containing a larger amount of cells other than erythrocytes and counting the number (P1) of resin particles P and the number (N1) of resin particles N contained in the layer.

In the present invention, both the counting of the number of resin particles P and the counting of the number of resin particles N in the Step (b) can be carried out in the same manner as described above in the section "Method of Counting Number of Cells of Interest or Number of Resin Particles".

In the Step (b), counting of the number of cells of interest may also be carried out, and the counting of the number of cells of interest may also be carried out in the same manner as described above in the section "[Method of Counting Number of Cells of Interest or Number of Resin Particles]".

[Step (c)]

Step (c) is a step of calculating (P1/P0)×100 and comparing the calculated value with a predetermined reference value to evaluate the extent to which the whole system functions. That is, Step (c) can also be regarded as a step of evaluating how much cells of interest for quantification can be extracted by the system to be evaluated, from the cells of interest contained in the blood-derived sample. For example, in cases where (P1/P0)×100 equals 100%, that is, in cases where P1=P0, the quantification result obtained by the system can be judged to be completely reflecting the amount of cells of interest contained in the blood-derived sample.

The "predetermined reference value" in Step (c) is not limited and may be set appropriately depending on the system. For example, the value may be set as follows.

In cases where (P1/P0)×100=90 to 100%, the system is judged to be sufficiently functioning; in cases where (P1/P0)×100=not less than 80% and less than 90%, the system is judged to be functioning almost without problems; in cases where (P1/P0)×100=not less than 70% and less than 80%, the system is judged to have a problem; and in cases where (P1/P0)×100=less than 70%, the system is judged to be only poorly functioning. Each judgment described above can be made depending on the range within which the (P1/PO) value actually calculated in Step (c) falls.

[Step (d)]

Step (d) is a step of calculating N1/P1 and comparing the calculated value with a predetermined reference value to evaluate the extent to which the layers are separated by the density gradient centrifugation.

The "predetermined reference value" in Step (d) is not limited and may be set appropriately depending on the system. For example, the value may be set as follows. In cases where N1/P1=less than 0.005, the isolation by density gradient centrifugation is judged to have no significant problems; and in cases where N1/P1=not less than 0.005, the isolation by density gradient centrifugation is judged to have a problem. Each judgment described above can be made depending on the range within which the N1/P1 value actually calculated in Step (d) falls.

[Step (e)]

Step (e), which may be arbitrarily carried out in the present invention, is a step of measuring an emission signal derived from the resin particles P in the number of P1 or the resin particles N in the number of N1, and calibrating an emission signal detector.

In the Step (e), the number (P1 or N1) of resin particles P or N can be counted by flow cytometry. The method of counting of resin particles P or N in the Step (e) is not limited to flow cytometry as long as the method also allows measurement of the amount of luminescence, and, if possible, the counting may be carried out by the two-dimensional method as described above in the section "Method of Counting Number of Cells of Interest or Number of Resin Particles".

By preliminarily measuring the amount of luminescence derived from the dye with which a single P or N resin particle is labeled, and comparing the value obtained by multiplying the amount of luminescence per particle by the number of corresponding resin particles with the value actually measured by a signal detector, the signal detector can be calibrated (adjusted so that the values would be consistent with each other). Therefore, the evaluation method of the present invention preferably further includes the Step (e).

<Kit>

The kit used for the quantification method contains a known number of the resin particles P, and the kit used for the evaluation method of the present invention contains known numbers of the resin particles P and the resin particles N.

These kits may also contain, in addition to the resin particles P, or the resin particles P and the resin particles N, a diluent or buffer for diluting the blood or the like used in the quantification method or evaluation method of the present invention; separation medium to be used for density gradient centrifugation; antibody conjugated with a fluorescent dye for fluorescent labeling of the cells of interest; and/or manufacturer's instruction, flow cytometer, fluorescence microscope, and/or computer for processing values obtained using these instruments.

EXAMPLES

The present disclosure is described below in more detail by way of Examples. However, the present invention is not limited by these.

[Predetermined Reference Value]

In the Examples below, the "predetermined reference value" was defined as follows.

* (P1/P0)×100 in Step (c):

90 to 100%: The system is sufficiently functioning.

Not less than 80% and less than 90%: The system is functioning almost without problems.

Not less than 70% and less than 80%: The system has a problem.

Less than 70%: The system is only poorly functioning.

* (N1/P1) in Step (d) :

Less than 0.005: The isolation by density gradient centrifugation has no significant problems.

Not less than 0.005: The isolation by density gradient centrifugation has a problem.

[Example 1]

To 2 mL of whole blood, 100 cultured MCF7 cells as a CTC model and 100 resin particles P labeled with fluorescein isothiocyanate (FITC) (FICP-80-2, manufactured by Spherotech, Inc.) (specific gravity, 1.050) (that is, P0=100) were added, and the resulting mixture was mixed, followed by performing density gradient centrifugation. More specifically, 2 mL of the whole blood was overlaid on 3 mL of Ficoll (specific gravity, 1.077) as a separation liquid, and centrifugation was carried out at 400xg for 40 minutes.

About 1.2 mL of the supernatant containing the cultured cells was extracted, and spread on the plane of a cell culture dish. A PE-labeled antibody (Anti EpCAM (manufactured by Beckton Dickinson)) was added to the culture dish to stain only the cultured cells with PE.

As a result of scanning fluorescence signals of PE and FITC with a detector, 68 PE labels (that is, the number of cultured cells after density gradient centrifugation=68) and 70 FITC labels (that is, P1=70) were detected. More specifically, the measurement of fluorescence signals were carried out by dropping the suspension on a 35-mm cell culture dish, leaving the dish to stand for several minutes, and then capturing a fluorescence image of the whole area in the dish using a fluorescence inverted microscope (Carl Zeiss, Observer D1) to judge the number of beads.

Multiplication of 100/70 as P0/P1 by the number of cultured cells after density gradient centrifugation was calculated as follows: $68 \times 100/70 = 97$. This represents an assumed number of cultured cells contained in 2 mL of the whole blood before density gradient centrifugation, and was almost the same as the number of cultured cells actually contained in the whole blood, 100.

[Example 2]

To 2 mL of whole blood, 100 cultured cells which were the same as those in Example 1 as a CTC model and 10,000 FITC-labeled resin particles P (that is, P0=10,000) were added, and the resulting mixture was mixed, followed by performing density gradient centrifugation in the same manner as in Example 1.

About 1.2 mL of the supernatant containing the cultured cells was extracted, and centrifuged at 400xg for 40 minutes to reduce the volume to 1 mL, followed by collecting 1/10 volume (=100 $\mu$L) of the obtained concentrate and spreading the collected concentrate on the plane of a cell culture dish.

By the same method as in Example 1, fluorescence of FITC was scanned with a detector. As a result, 990 FITC labels (that is, $P1 \times 1/10 = 990$) were detected.

$(P1/P0) \times 100$, which is an index indicating the extent to which the whole system is functioning, was calculated as follows: $[(990 \times 10)/10,000] \times 100 = 99\%$. Since this was a significantly higher value than the predetermined reference value, the system could be judged to be sufficiently functioning.

[Example 3]

To 2 mL of whole blood, 100 cultured cells which were the same as those in Example 1 as a CTC model, 10,000 FITC-labeled resin particles P (that is, P0=10,000), and 10,000 DMEQ-hydrazide-labeled resin particles N (prepared by reacting BM30X-5 given carboxyl groups manufactured by Sekisui Plastics Co., Ltd.

with DMEQ-hydrazide (Wako Pure Chemical Industries, Ltd.)) (specific gravity, 1.10) as an erythrocyte model (that is, N0=10,000) were added, and the resulting mixture was mixed, followed by performing density gradient centrifugation in the same manner as in Example 1.

About 1.2 mL of the supernatant containing the cultured cells was extracted, and centrifuged to reduce the volume to 1 mL, followed by collecting 1/10 volume (=100 $\mu$L) of the obtained concentrate and spreading the collected concentrate on the plane of a cell culture dish.

By the same method as in Example 1, fluorescence of each of FITC and DMEQ-hydrazide was scanned with a detector. As a result, 990 FITC labels (that is, $P1 \times 1/10 = 990$) and 2 DMEQs (that is, $N1 \times 1/10 = 2$) were detected.

N1/P1, which is an index indicating the extent to which the layers are separated by the density gradient centrifugation, was calculated as follows: $(2 \times 10)/(990 \times 10) = 0.002$. Since this falls within the range of the predetermined reference value, the isolation by density gradient centrifugation could be judged to have no significant problem.

In the present Example, $(P1/P0) \times 100$ can be calculated as follows: $[(990 \times 10)/10,000] \times 100 = 99\%$.

[Comparative Example 1]

In Comparative Example 1, quantification of CTCs was carried out using, as an internal reference, stabilized cells instead of the resin particles P.

To 2 mL of whole blood, 100 cultured cells which were the same as those in Example 1 as a CTC model, and 100 cultured cells which were the same as those in Example 1 and were subjected to special treatments (paraformaldehyde treatment and FITC staining) as an internal control were added, and the resulting mixture was mixed, followed by performing density gradient centrifugation in the same manner as in Example 1.

The supernatant containing the cultured cells was extracted, and spread on a plane. A fluorescent dye that stains only cultured cells (Alexa Fluor 647) was added to the cells.

As a result of scanning fluorescence signals of Alexa Fluor 647 and FITC with a detector, 69 Alexa-Fluor-647 labels (that is, the number of cultured cells after density gradient centrifugation=69) and 20 FITC labels (that is, the number of particles for internal control after density gradient centrifugation=20) were detected.

Multiplication of 100/20, which is (the number of particles for internal control before density gradient centrifugation)/(the number of particles for internal control after density gradient centrifugation), by the number of cultured cells after density gradient centrifugation was calculated similarly to Example 1 as follows: $69 \times 100/20 = 345$. This represents an assumed number of cultured cells contained in 2 mL of the whole blood before density gradient centrifugation, and was largely different from the number of cultured cells actually contained in the whole blood, 100.

DESCRIPTION OF SYMBOLS

1 ... Specific-gravity liquid
2 ... Blood-derived sample
3 ... Resin particle P, which has a specific gravity larger than a specific gravity of blood plasma but smaller than a specific gravity of erythrocytes
4 ... Layer containing a larger amount of erythrocytes
5 ... Layer containing a larger amount of cells other than erythrocytes

**Claims**

1. An evaluation method for evaluating the reliability of a system for quantifying a cell(s) of interest that is/are potentially contained in a blood-derived sample (2) and has/have a specific gravity larger than 1.025 but smaller than 1.120, said method comprising the Steps (a) to (d) below:

   (a) separating a blood-derived sample (2) containing a known number, P0, of resin particles P (3) having a specific gravity larger than 1.025 but smaller than 1.120 and a known number, N0, of resin particles N having a specific gravity of not less than 1.090 and not more than 1.120 by density gradient centrifugation into at least two layers including a layer (4) containing a larger amount of erythrocytes and a layer (5) containing a larger amount of cells other than erythrocytes;
   (b) extracting said layer (5) containing a larger amount of cells other than erythrocytes and counting the number, P1, of resin particles P (3) and the number, N1, of resin particles N contained in said layer (5);
   (c) calculating $(P1/P0)\times100$ and comparing the calculated value with a predetermined reference value to evaluate the extent to which the whole system functions; and
   (d) calculating N1/P1 and comparing the calculated value with a predetermined reference value to evaluate the extent to which the layers are separated by said density gradient centrifugation.

2. The evaluation method according to claim 1, wherein said resin particles P are formed from a water-insoluble resin and labeled with an optically detectable dye Da.

3. The evaluation method according to claim 1 or 2, wherein said resin particles P have a specific gravity of not less than 1.040 and not more than 1.085.

4. The evaluation method according to claim 1 or 2, wherein said resin particles P have a specific gravity of not less than 1.040 and not more than 1.077.

5. The evaluation method according to any one of claims 1 to 4, wherein said resin particles N are formed from a water-insoluble resin and labeled with an optically detectable dye Db having an emission wavelength different from the emission wavelength of the optically detectable dye Da.

6. The evaluation method according to any one of claims 1 to 5, wherein said cell(s) of interest is/are at least one type of rare cell(s) selected from the group consisting of circulating tumor cells, CTCs, circulating stem cells and circulating endothelial cells.

7. The evaluation method according to any one of claims 1 to 6, further comprising the Step (e) below: (e) measuring an emission signal derived from said resin particles P in the number of P1 or said resin particles N in the number of N1, and calibrating an emission signal detector.

**Patentansprüche**

1. Bewertungsverfahren zur Bewertung der Zuverlässigkeit eines Systems zur Quantifizierung einer/von Zelle(n) von Interesse, die möglicherweise in einer aus Blut gewonnen Probe (2) enthalten ist/sind, und die eine spezifische Dichte von größer als 1,025, aber kleiner als 1,120 aufweist/aufweisen, wobei das Verfahren die unten genannten Schritte (a) bis (d) aufweist:

   (a) Trennen einer aus Blut gewonnen Probe (2), die eine bekannte Anzahl, P0, an Harzpartikeln P (3) mit einer spezifischen Dichte von größer als 1,025, aber kleiner als 1,120 und eine bekannte Anzahl, N0, an Harzpartikeln N mit einer spezifischen Dichte von nicht weniger als 1,090 und nicht mehr als 1,120 aufweist, durch Dichtegradienten-Zentrifugation in zumindest zwei Schichten mit einer Schicht (4), die eine größere Menge von Erythrozyten aufweist, und einer Schicht (5), die eine größere Menge von anderen Zellen als Erythrozyten aufweist:
   (b) Extrahieren der Schicht (5), die eine größere Menge von anderen Zellen als Erythrozyten aufweist, und Zählen der Anzahl P1 an Harzpartikeln P (3) und der Anzahl N1 an Harzpartikeln N, die in der Schicht (5) enthalten sind;
   (c) Berechnen (P1/P0)x 100 und Vergleichen des berechneten Wertes mit einem vorab bestimmten Referenzwert, um den Umfang zu bewerten, in welchem das gesamte System funktioniert; und

(d) Berechnen N1/P1 und Vergleichen des berechneten Wertes mit einem vorab bestimmten Referenzwert, um den Umfang zu bewerten, in welchem die Schichten durch die Dichtegradienten-Zentrifugation voneinander getrennt worden sind.

2. Bewertungsverfahren gemäß Anspruch 1, wobei die Harzpartikel P aus einem wasserunlöslichen Harz gebildet und mit einem optisch erfassbaren Farbstoff Da gekennzeichnet sind.

3. Bewertungsverfahren gemäß Anspruch 1 oder 2, wobei die Harzpartikel P eine spezifische Dichte von nicht weniger als 1,040 und nicht mehr als 1,085 aufweisen.

4. Bewertungsverfahren gemäß Anspruch 1 oder 2, wobei die Harzpartikel P eine spezifische Dichte von nicht weniger als 1,040 und nicht mehr als 1,077 aufweisen.

5. Bewertungsverfahren gemäß irgendeinem der Ansprüche 1 bis 4, wobei die Harzpartikel N aus einem wasserunlöslichen Harz gebildet und mit einem optisch erfassbaren Farbstoff Db gekennzeichnet sind, der eine andere Emissionswellenlänge als die Emissionswellenlänge des optisch erfassbaren Farbstoffs Da aufweist.

6. Bewertungsverfahren gemäß irgendeinem der Ansprüche 1 bis 5, wobei die Zelle(n) von Interesse zumindest eine Art von seltener(n) Zelle(n) ist/sind, die aus der Gruppe ausgewählt ist/sind, die aus zirkulierenden Tumorzellen, CTCs, zirkulierenden Stammzellen und zirkulierenden Endothelzellen besteht.

7. Bewertungsverfahren gemäß irgendeinem der Ansprüche 1 bis 6, mit ferner dem unten stehenden Schritt (e):

(e) Messen eines Emissionssignals, das aus den Harzpartikeln P in der Anzahl P1 oder den Harzpartikeln N in der Anzahl N1 stammt, und Kalibrieren eines Emissionssignaldetektors.

**Revendications**

1. Procédé d'évaluation pour évaluer la fiabilité d'un système destiné à quantifier une cellule/des cellules d'intérêt, qui est/sont potentiellement contenue(s) dans un échantillon dérivé du sang (2) et qui comprend/comprennent une gravité spécifique plus grande que 1,025, mais plus faible que 1,120, ledit procédé comprenant les étapes (a) à (d) ci-après de:

(a) diviser un échantillon dérivé du sang (2) contenant un nombre connu, P0, de particules de résine P (3) ayant une gravité spécifique plus grande que 1,025, mais plus faible que 1,120 et un nombre connu, N0, de particules de résine N ayant une gravité spécifique d'au moins 1,090 et d'au plus de 1,120, en au moins deux couches comprenant une couche (4), qui contient une quantité supérieure d'érythrocytes, et une couche (5), qui contient une quantité supérieure de cellules différentes d'érythrocytes, par moyen de la centrifugation par gradient de densité ;

(b) extraire ladite couche (5), qui contient une quantité supérieure de cellules différentes d'érythrocytes, et calculer le nombre, P1, de particules de résine P (3) et le nombre, N1, de particules de résine N contenues dans ladite couche (5) ;

(c) calculer (P1/P0)x 100 et comparer la valeur calculée avec une valeur de référence prédéterminée pour évaluer la mesure, dans laquelle fonctionne le système entier ; et

(d) calculer N1/P1 et comparer la valeur calculée avec une valeur de référence prédéterminée pour évaluer la mesure, dans laquelle les couches sont séparées par moyen de ladite centrifugation par gradient de densité.

2. Procédé d'évaluation selon la revendication 1, dans lequel lesdites particules de résine P sont formées à partir d'une résine insoluble dans l'eau et marquées par un colorant Da optiquement détectable.

3. Procédé d'évaluation selon la revendication 1 ou la revendication 2, dans lequel lesdites particules de résine P ont une gravité spécifique d'au moins 1,040 et d'au plus 1,085.

4. Procédé d'évaluation selon la revendication 1 ou la revendication 2, dans lequel lesdites particules de résine P ont une gravité spécifique d'au moins 1,040 et d'au plus 1,077.

5. Procédé d'évaluation selon l'une quelconque des revendications 1 à 4, dans lequel lesdites particules de résine N sont formées à partir d'une résine insoluble dans l'eau et marquées par un colorant Db optiquement détectable, qui comprend une longueur d'onde d'émission, qui diffère de la longueur d'onde d'émission du colorant Da optiquement détectable.

6. Procédé d'évaluation selon l'une quelconque des revendications 1 à 5, dans lequel la/les dite(s) cellule(s) d'intérêt est/sont au moins un type de cellule(s) rare(s) sélectionnée(s) dans le groupe composé de cellules tumorales circulantes, les CTCs, de cellules souches circulantes et de cellules endothéliales circulantes.

7. Procédé d'évaluation selon l'une quelconque des revendications 1 à 6, comprenant en outre l'étape (e) ci-après de :

   (e) mesurer un signal d'émission dérivé desdites particules de résine P dans le nombre de P1 ou desdites particules de résine N dans le nombre de N1 et calibrer un détecteur de signal d'émission.

[Fig. 1]

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2010248257 A1
- JP 2004534210 W
- EP 0958046 A1
- WO 9738313 A1
- JP 2010169519 A
- JP H4252957 A